# EUROPÄISCHE PATENTSCHRIFT

(11) **EP 2 099 396 B1**
(45) Veröffentlichungstag und Bekanntmachung des Hinweises auf die Patenterteilung: **26.06.2013**
(21) Anmeldenummer: 07846365.0
(22) Anmeldetag: 16.11.2007
(51) Int. Cl.: A61F 5/41, A41B 9/02

(54) **KLEIDUNGS-INTEGRIERTES PENISEXTENSIONSSYSTEM**
PENIS EXTENSION SYSTEM INTEGRATED INTO AN ITEM OF CLOTHING
SYSTÈME D'EXTENSION DE PÉNIS INTÉGRÉ À UN VÊTEMENT

(30) Priorität: 16.11.2006 DE 102006054642; 09.03.2007 DE 102007012319; 23.04.2007 DE 102007020236
(43) Veröffentlichungstag der Anmeldung: 16.09.2009
(73) Patentinhaber: ETTMER, Jürgen, 30177 Hannover (DE)
(72) Erfinder: ETTMER, Jürgen, 30177 Hannover (DE)
(86) Internationale Anmeldenummer: PCT/DE2007/002115
(87) Internationale Veröffentlichungsnummer: WO 2008/058532

(56) Entgegenhaltungen:
- WO-A-2007/084022
- CA-A1- 2 444 663
- DE-U1- 29 718 299

## Beschreibung

Die Erfindung betrifft ein in die Bekleidung eines Benutzers integrierbares, mobil zu tragendes Penisextensionssystem zur Vergrößerung des Penis und der Eichel.

### Stand der Technik

Vorrichtungen zum dauerhaften Strecken menschlicher Körperteile sind in verschiedenen Ausführungen bekannt und ihre Wirksamkeit wurde in klinischen Tests nachgewiesen.

Die Patentschrift CA 2 444 663 stellt den nächsliegenden Stand der Technik das. Weitere veröffentlichte Vorrichtungen zur Streckung und dauerhaften Verlängerung des Penis sind beispielsweise:
1. WO1996026691 A1
2. W02003073967 A 1
3. DE19915407 A1
4. WO1999018897 A1
5. DE 10001331 A1

1. WO1996026691 A1 ist eine Vorrichtung deren Dehnelement durch zwei Stangen gebildet wird, die auf einem die Peniswurzel umschließenden Stützring gelagert sind und durch deren Einstellung in der Länge ein Dehnungsdruck zwischen der Peniswurzel und der mit einer Befestigungseinrichtung am anderen Ende der Stangen befestigten Eichel erzeugt wird. Die Vorrichtung lässt sich nicht unauffällig unter der Kleidung tragen und birgt zusätzlich gesundheitliche Risiken für den Benutzer. Die Gerätelänge während des Gebrauchs ergibt sich durch die Länge des erschlafften, zu streckenden Penis. Dieser hat im Mittel eine Länge von 7-10 cm. Das Gerät drückt also, wenn es tatsächlich unter der Kleidung getragen wird, mit der die rechte oder die linke Metallstange schützenden Kunststoffecke der in sich starren Vorrichtung, in einem Abstand von 7-10 cm von der Peniswurzel entfernt, auf die hier verlaufenden wichtigen Blutgefäße wie Bein- und Beckenarterie oder den Venenstern. Die Stützstangen bergen bei falschem Sitz der Vorrichtung das Risiko auf die Hoden zu drücken und eine Hadenquetschung zu verursachen Durch die in den Stützstangen gelagerten Federn ist lediglich ein Nachgeben von circa 5 mm der Zugvorrichtung gewährleistet. Dies ist für ein schmerzfreies Hinsetzen und unvermittelte Bewegungen des Benutzers absolut nicht ausreichend, da der mit Sehnen im Inneren des Körpers befestigte Penis schon beim Hinsetzen einen Spielraum von mindestens zwei Zentimetern beaötigt.
   Ein dauerhaftes, ganztägiges Tragen dieses nicht an den anatomischen Gegebenheiten ausgeformten Fremdkörpers ist während einer durchschnittlichen Beschäftigung aus diesen Gründen nicht möglich, wodurch ein Erfolg, der nur über eine dauerhafte Streckung erreicht werden kann, fraglich ist.
2. W02003073967 A1 ist nach dem gleichen Wirkprinzip wie die vorstehend beschriebene Vorrichtung WO1996026691 A1 konstruiert, so dass die dort bemängelten Merkmale auch auf diese Vorrichtung zutreffen. Zusätzlich weist das Spannelement (18 der Veröffentlichung) zwei feste, beim Tragen unter der Kleidung, in den Unterleib des Anwenders drückende Zuglaschen (18d der Veröffentlichung) auf.
3. DE19915407 A1 streckt den Penis, indem ein Benutzer den Penis durch die Öffnung einer Grundplatte, entlang einer auf der Grundplatte befestigten Hubsäule führt und die Penisspitze an einer an der Hubsäule verschiebbaren Zugfixierebene befestigt.
   Die Anwendung ist insofern gefährlich, dass bei einer plötzlichen Bewegung des Benutzers verletzungsrelevante Zugkräfte auf den Penis wirken und ihn schädigen können, da die Fixierebene der Eichel mit einer Schraube an der Hubsäule befestigt ist. Aus diesem Grund ist es dem Benutzer auch nicht möglich, sich während der Anwendung zu setzen oder zu bücken. Da der Penis im Inneren des Körpers mit den Beckenknochen verbunden ist, muss eine Streckungsvorrichtung ein Nachgeben gewährleisten können. Auch diese Vorrichtung lässt sich nicht unauffällig unter der Kleidung tragen.
4. WO1999018897 A1
   Als einzige bekannte Vorrichtung ist diese unauffällig unter der Bekleidung zu tragen. Ein flexibles, längenverstellbares Dehnelement mit einem als Widerlager für die Peniswurzel dienenden Ring wird um den Körper des Benutzers geschlungen und auf der anderen Seite an der Eichel des Benutzers befestigt. Die dadurch entstehenden Zugkräfte führen zu einer allmählichen Verlängerung des Penis.
   Ein großer Nachteil der Anwendung ist die Befestigung des zwischen Eichel und Peniswurzel verlaufenden Dehnelements an der Eichel mittels einer Schlaufenkonstluktion. Die Blutzufuhr zur Eichel wird entsprechend der Zugkraft abgeschnürt, es kommt zu Durchblutungsstörungen und im schlimmsten Fall zum Absterben der Eichel. Darüber hinaus wird die Eichel durch die hebelartig Schlaufenbefestigung entgegen ihrer normalen Stellung zum Peniskörper, nach oben gedrückt, was bei langzeitigem Gebrauch zu Veränderungen des Penis und damit verbundenen Schädigungen fuhren könnte.
   Das Anlegen der Vorrichtung ist mit einem hohen Aufwand verbunden, da das um den Körper zu schlingende Dehnelement durch seine konstruktionsbedingte Zugeigenschaft ständig unter Spannung steht, so dass das Befestigen der Eichel einiges Geschick erfordert.

Die weiterentwickelte Vorrichtung der vorstehend behandelten WO1999018897 A1 ist von der Streckungsfunktion gleich, weist jedoch als Befestigungselement für die Eichel statt der Schlingenkonstruktion, ein weiches, überstülpbaren Hütchen auf welches sich an die Eichel anschmiegen soll. Mittels Unterdruck soll die Eichel in diesem Hütchen gehalten werden und wie in WO1999018897 A1 mit dem um den Körper geschlungenen Dehnelement verbunden werden. Diese Befestigung der Eichel funktioniert nicht. Die Eichel zieht sich, da sie keinen festen Halt hat, nach kurzer Zeit aus diesem Hütchen weit unter die Penishaut zurück, wodurch lediglich diese durch den Unterdruck gehalten, langgezogen und überdehnt wird. An der Eichel kann es hierdurch und durch langes Aufrechterhalten des Unterdrucks zu Taubheitsgefühlen kommen, die Penishaut wird schlaff und faltig, während eine Streckung des eigentlichen Penis garnicht stattfindet.

Alle erwähnten gattungsmäßigen Penisextensionsgeräte haben den Mangel, dass die Eichel schon nach kurzer Streckdauer aufgrund ungenügender Durchblutung kalt werden und eine bläuliche Verfärbung annehmen kann. Die zur Fixierung des distalen Endes des Penis strangulierte Eichel entwickelt sich in Größe und Umfang aufgrund dieser Behandlung nicht proportional und bleibt im Wachstum zurück. Bei allen Vorrichtungen handelt es sich um in sich geschlossene Apparaturen mit in sich geschlossenen Kraftkreisläufen zur Installation am nackten männlichen Körper.

Ihre Funktionsfähigkeit ist hergestellt, wenn einem Objektträger an der Eichel ein Widerlager an der Peniswurzel entgegensteht und durch ein beliebiges Dehnelement eine gegeneinander gerichtete Zugkraft erzeugt wird. Dadurch, dass Zug und Gegenzug durch das verbindende Dehnelement unmittelbar und in Abhängigkeit voneinander an beiden Enden des Penis wirksam werden, besteht die Gefahr eines folgenschweren Unfalls. Im ungünstigsten Fall kann der mit Sehnen im Beckenboden verankerte Penis ausgerissen oder schwer verletzt werden.

Ein Penisextensionssystem mit Widerlager ist aus der DE 297 18299U bekannt.

Eine schematische Darstellung der Kraftkreisläufe der gegenübergestellten Vorrichtungen offenbart deren identische Funktionsweise bestehend aus drei, jeweils dem gleichen Zweck dienenden statischen Elementen:
1. Befestigungsvorrichtung für die Eichel
2. verbindendes Dehnelement
3. Widerlager für die Peniswurzel.

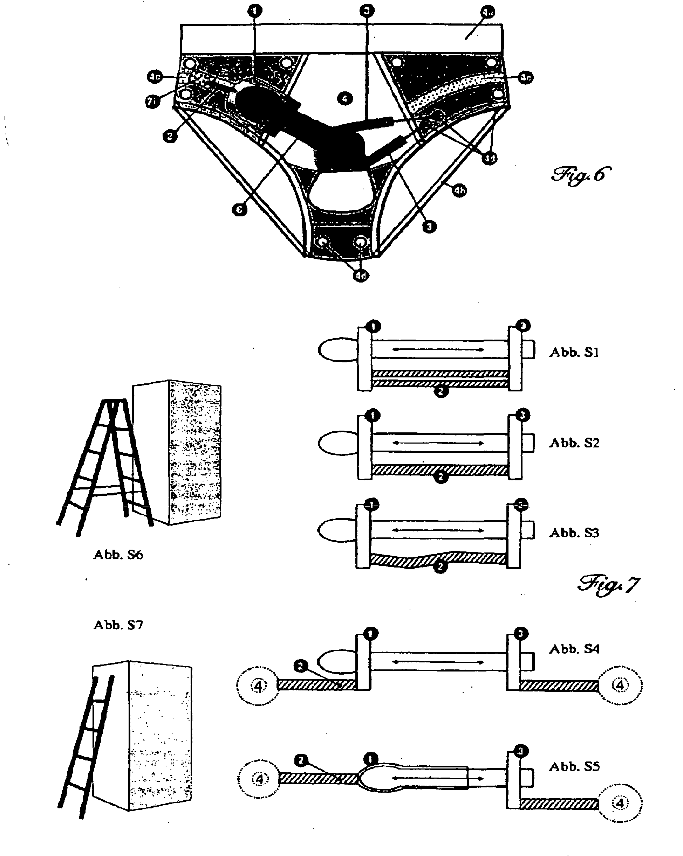

Die Vorrichtungen unterscheiden sich von ihrer Wirkweise lediglich durch unterschiedliche Dehnelemente. WO1996026691 A1 und W02003073967 A1 (Fig. 7, Abb. S1) verwenden als Dehnelement zwei parallel verlaufende Stangen. DE19915407 A1 (Fig. 7, Abb. S2) verwendet als Dehnelement eine Hubsäule, während bei WO1999018897 A1 und DE 10001331 A1 (Fig. 7, Abb. S3) das Dehnelement als Band ausgebildet ist.

Die Einstellung der auf den Penis wirkenden Zugkraft geschieht indirekt, sie kann von einem unbeschlagenen Benutzer nur abstrakt wahrgenommen und eingeschätzt werden. Ebenfalls ist auch das Anlegen aller Vorrichtungen sehr umständlich und nur unter zu Hilfenahme beider Hände möglich.

Allen Anwendungen liegt der Gedanke zu Grunde, dem Benutzer eine Vorrichtung zur Verfügung zu stellen, in die er nur noch seinen Penis einspannen muss, der dann gestreckt und gedehnt wird. Dieser abstrakte Anspruch erklärt die Umständlichkeit jener Erfindungen und berücksichtigt nicht den Umstand, dass es sich hier um den empfindlichsten Körperteil des Mannes handelt.

Aus den vorstehend beschriebenen Nachteilen der bekannten gattungsmäßigen Penisextensionsgeräte ergab sich die Aufgabenstellung für die vorliegende Erfindung.

### Aufgabenstellung

Die ursprüngliche, einfache Penisstreckung ist das Langziehen mit der Hand. Ein Benutzer sieht und erlebt den Zug unmittelbar und zieht intuitiv mit der richtigen Stärke.

Aufgabe eines Streckungsgerätes muss es also sein, dieses intuitive, für jeden Mann individuelle Wissen seiner Belastbarkeit für die Anwendung nutzbar zu machen. Skalen mit Newton oder Pond auf den Vorrichtungen werden dieser individuellen Empfindsamkeit nicht gerecht und sind deshalb nur begrenzt anwendbar.

Die vorliegende Erfindung geht deshalb von der Aufgabe aus, den Penis direkt und unmittelbar mit der Hand durch die Muskelkraft des am Körper befindlichen Armes lang zu ziehen und in diesem gestreckten Zustand zu halten, dabei eventuellen Gegenzug durch Bewegungen wie Hinsetzen oder andere körperliche Betätigungen, flexibel ausgleichen zu können.

Dieser körperliche Bewegungsablauf soll nun durch die vorliegende Erfindung ersetzt werden. Da kein Mann seinen Penis an der Eichel langziehen wird, muss der Zug am Penisschaft ansetzen und die empfindliche Eichel schonen. Die Vorrichtung soll vor allem schmerzfrei und ohne gesundheitliche Gefährdungen ganztags während einer üblichen Tagesarbeit unauffällig anwendbar sein.

### Art der Anwendung:

Die vorliegende Erfindung löst diese Aufgabe gemäß den Merkmalen des Patentanspruchs 1 mit einer Vorrichtung bei der, bildlich formuliert, die Hand durch ein Befestigungselement für den Penisschaft, der Arm durch ein Zugmedium und die Schulter durch eine Befestigungsvorrichtung ersetzt wird. Der Körper als Basis für die Befestigung, wird durch die Bekleidung eines Benutzers ersetzt, an welcher unabhängig voneinander sowohl das Zugelement, wie auch ein Widerlagerelement befestigt werden können. Eine schematische Darstellung des Kraftkreislaufes (Fig.7, Abb. S4 und S5), veranschaulicht im Vergleich mit den gegenüber gestellten Anwendungen, die Vorteile eines vierten, externen statischen Elements, des Basiselements, das aktiv in den Kraftkreislauf eingebunden werden kann und einem Benutzer das bequeme Befestigen eines oder beider unabhängiger Zugelemente in unterschiedlichsten Richtungen mit den unterschiedlichsten Zugstärken und Anwendungen erlaubt.
1a. Befestigungsvorrichtung für den Penis
2a. separates Zugelement für den Penis
3a. separates Widerlager für die Peniswurzel.
4a. externe Basiselemente zur Befestigung des Zugelements und des Widerlagerelements

Ein Benutzer zieht die Unterhose, die in diesem Fall das Basis- oder Bekleidungselement darstellt, an. Anschließend befestigt er den Penis in dem Eichelschlitten, der nicht mit dem Basiselement sondern mit dem Zugelement verbunden ist. Nun entscheidet er sich, in welche Richtung er den Penis streckend lagern möchte und zieht das Zugelement in die gewünschte Richtung. Bei Erreichen einer ihm angenehmen Zugesstärke befestigt er das Zugelement mit Hilfe der Befestigungsvorrichtung an einer Stelle seiner Unterhose oder Bekleidung. Die Befestigungsvorrichtung ist mit der, einem Benutzer während der Anwendung zugewandten Seite gepolstert, um einen punktuellen Druck auf seine Haut zu vermeiden. Desweiteren kann der Benutzer bei Bedarf das nicht mit dem Zugelement verbundene Widerlagerelement um die Peniswurzel oder um Peniswurzel und Hodensack zusammen legen, und in der, der Zugrichtung an der Penisspitze gegenüberliegenden Zugrichtung, mit Hilfe der Befestigungsvorrichtung des Widerlagerelementes, an einer entsprechenden Stelle der Unterhose oder Bekleidung spannen und befestigen, wodurch ein Widerlager gegen die an der Penisspitze wirkende Zugkraft entsteht und ein intensiver Zug auf den Peniskörper einwirkt.

Nun schließt er nur noch seine Oberbekleidungshose, die auch eine enge Jeans sein kann. Es ist ihm nun möglich, diese Vorrichtung den ganzen Tag unauffällig zu tragen und sich ungehindert zu bewegen.

Die Erfindung erfüllt die in der Aufgabe definierten Forderungen und stellt gegenüber den bestehenden Vorrichtungen einen großen Fortschritt dar. Sie ist kein abstrakt konstruierter zusätzlicher Fremdkörper, sondern fügt sich unauffällig als Unterhose ausgestaltet, ähnlich dem vor 100 Jahren erfundenen BH, der bei den Frauen ebenfalls formgebende Aufgaben erfüllt, in die anatomischen Gegebenheiten und in die üblichen Bekleidungsgewohnheiten von Männern ein.

Zwei unabhängige Zugelemente lassen sich je nach Wunsch des Anwenders an jeder beliebigen Stelle seiner Bekleidung befestigen und in eine Vielzahl von Richtungen spannen oder ziehen. Da zwischen Zugelement und Widerlagerelement keine direkte Verbindung und Kraftübertragung besteht, sind Unfälle durch willkürlich und plötzlich entstehende Krafteinwirkung ausgeschlossen.

Es handelt sich im Gegensatz zu den erwähnten geschlossenen Systemen, um ein offenes System, welches externe Befestigungsmöglichkeiten wie zum Beispiel die Kleidung, als Befestigungsbasis nutzt. Als solches stellt die Vorrichtung in diesem Marktsegment eine Neuheit dar und grenzt sich durch ihre Funktionsweise von herkömmlichen, in sich geschlossenen Geraten ab.

Zum besseren Verständnis stelle man sich eine Stehleiter vor, die ein in sich funktionierendes, geschlossenes, nicht integriertes System zum Überwinden von Höhenunterschieden darstellt. (Fig. 7, Abb. S6) Die vorliegende offene Vorrichtung ist mit einer Anstell-Leiter vergleichbar, die sich in vorhandene externe Gegebenheiten integriert und sich diese als Befestigungsbasis zum Überwinden eines Höhenunterschiedes nutzbar macht Sie ist dadurch leicht zu tragen und auf kleinster Grundfläche vielseitig einsetzbar. (Fig. 7, Abb. S6)

Einem Benutzer ist es möglich, sich während der Anwendung zu setzen oder zu bücken. Der im Inneren des Körpers durch Sehnen mit den Beckenknochen verbundene Penis wird der Gefahr eines Sehnen- oder Geweberisses nicht ausgesetzt, da das flexible Zugelement in Verbindung mit der von ihrer stofflichen Beschaffenheit ebenfalls flexiblen Bekleidung, trotz fest eingestelltem Zug auf den Peniskörper, ausreichend Spielraum für ein Nachgeben von mindestens 6 cm gewährleistet.

Die Irrtegration eines Penisextensionssystems in die übliche Bekleidung eines Benutzers vervielfacht im Vergleich zu allen anderen, in sich geschlossenen Extensionsvorrichtungen die statischen Haltepunkte um ein Vielfaches, da beide Beine und der Unterkörper eines Benutzers hierzu das authentischste, weil anatomisch vorgegebene, Fundament bilden.

Ein weiterer Anwendungsvorteil besteht darin, dass die Vorrichtung mit nur einer Hand gespannt und befestigt und wieder gelöst werden kann.

Auch der Gang zur Toilette gestaltet sich problemlos. Der Benutzer öffnet mit einer Hand den Klettverschluss am Eichelschlitten und kann sein Geschäft erledigen. Zugelement und Widerlagerelement sind weiterhin mit der Hose verbunden und können nicht herunterfallen.

Ergänzend zu den vorstehend dargestellten Vorteilen im Vergleich zum gattungsmäßigen Stand der Technik zeichnet sich das erfindungsgenläße Penisextensionssystem durch die besonders schonende Penisbefestigung aus. Durch die halbkreisförmige Umschließung des Penis mit dem halbzylindermantelförmigen Eichelschlitten und dem breitflächigen Verschlussriemen wirkt die zur Befestigung notwendige Kraft nicht strangulierend, sondern verteilt sich auf eine große Fläche des vorderen Penisschaftes, wodurch die Durchblutung der Eichel nach dem Prinzip sogenannter Erektionsringe gefördert und eine Minderdurchblutung der Eichel vermieden wird. Insbesondere das Hinsetzen und die ungehinderte sportliche und berufliche Betätigung während der Anwendung werden hierdurch schmerzlos möglich.

Auch die besondere Berücksichtigung einer schonenden und durchblutungsfördernden Behandlung der Eichel durch den alternativ als Befestigungselement zu verwendenden Unterdruckbehälter ist neu und bietet erstmals eine intensive Streckung des Peniskörpers bei gleichzeitiger intensiver umfänglicher Volumen-Ausdehnung von Penis und Eichel, um deren proportionales Wachstum zueinander zu gewährleisten. Auch diese Anwendung ist zwar unter der Kleidung zu tragen, trägt jedoch so sehr auf, dass sich das Tragen eher auf den häuslichen Bereich beschränken sollte.

Nur durch eine möglichst lange tägliche Dehnungsbehandlung ist eine tatsächliche Vergrößerung des Penis und der Eichel zu erreichen. Eine solche langzeitige Anwendung ist mit dieser Vorrichtung aufgrund der kleinen und zueinander flexiblen, unabhängig in die Bekleidung integrierten Bestandteile über den ganzen Tag während einer üblichen körperlichen Beschäftigung unauffällig in der Öffentlichkeit möglich und kann zudem um die ergänzenden, aufeinander abgestimmten Anwendungskomponenten wie Unterdruckzylinder und Gewichtstraining im häuslichen Bereich erweitert werden.

### Bevorzugte Ausführungsformen

Eine erste bevorzugte Weiterbildung der erfindungsgemäßen Penisextensionsvorrichtung stellt sich wie folgt dar Ein Benutzer legt seinen Penis in den mit dem Zugelement verbundenen, halbzylindermantelförmigen Eichelschlitten, entlang dessen Längsachse und befestigt ihn an diesem hinter der Eichel indem er einen, quer zur Längsachse des zylindermantelförmigen Eichelschlitten, dessen Radius folgend, am Eichelschlitten befestigten Klettverschlussriemen hinter der Eichel über den Penisschaft und die gegenüberliegende Längsseite des Eichelschlitten zieht und auf der dem Penis abgewandten Außenseite des zylindermantelförmigen Eichetschlittens an der hier befindlichen Flauschseite des Klettbandes, durch Andrücken befestigt. Nun nimmt er das andere Ende des Zugmediums, an dem sich die Befestigungsvorrichtung, in diesem Fall ein an seiner dem Körper eines Benutzers zugewandten Unterseite, gepolsterter Hosenträgerclip, für die Kleidung befindet und befestigt diese Befestigungsvorrichtung mit einer ihm angenehmen Zugstärke an einer entsprechenden Stelle seiner Unterhose oder einer anderen ihm passend erscheinenden Stelle seiner Bekleidung.

Es ist ihm nun möglich, diese Vorrichtung den ganzen Tag unauffällig zu tragen und sich ungehindert zu bewegen.

Um eine noch intensivere Zugkraft auf den Peniskörper auszuüben, kann ein Benutzer bei Bedarf ein nicht mit der Zugvorrichtung verbundenes separates Widerlager in Form einer gepolsterten, dehnbaren Schlaufe, die um die Peniswurzel oder um Peniswurzel und Hodensack zusammen gelegt wird, in die, der Zugrichtung an der Penisspitze gegenüberliegenden Zugrichtung spannen und mit der Befestigungsvorrichtung für das Widerlager, in diesem Fall ein gewebeschonender, an seiner Unterseite gepolsterter Hosenträgerclip, an seiner Unterhose oder Hose befestigen wodurch der Peniskörper einer andauernden, jedoch nachgebenden und flexiblen Zugspannung ausgesetzt ist.

Eine weitere bevorzugte Weiterbildung der erfindungsgemäßen Penisextensionsvonichtung besteht darin, dass das Zugelement elastisch dehnbar ist. Der dehnbare Zug verbessert den Tragekomfort und die Bewegungsfreiheit für einen Benutzer und gewährleistet dabei ein permanentes Strecken des Penis.

Eine andere bevorzugte Weiterbildung der Vorrichtung besteht darin, dass das mit dem Befestigungselement für den Penis verbundene Zugelement austauschbar ist. So hat ein Benutzer die Möglichkeit, Gummibänder mit unterschiedlicher Zugstärke und Elastizität oder auch nicht dehnbare Medien zu verwenden oder ein **zusätzlich** in seiner Länge verstellbares Zugelement zu benutzen.

Eine weitere bevorzugte Weiterbildung der Vorrichtung besteht darin, dass die Befestigungsvorrichtung zur Befestigung des Zugelementes an der Kleidung auch als Klettverschluss ausgebildet sein kann, dessen Gegenstück an der Unterhose eines Benutzers befestigt ist, so dass dieser das Zugmedium durch Andrücken befestigen kann. Hier kommt auch ein bei Textilien oft benutzter Druckknopfverschluss oder ein einfacher gängiger **Knopfverschluss** in Frage. Ebenfalls denkbar ist die Befestigung des Zugmediums mit einem Haken- oder Ösenverschluss wie er häufig für **BH's** verwendet wird. Eine andere, aufwendigere Variante sieht die Verwendung eines Schnell- oder Clipverschlusses mit Gegenstück vor, welcher durch Zusammendrücken mit zwei Fingern lösbar ist. Auch denkbar ist die Verwendung eines Verschlusses ähnlich dem eines wieder verwendbaren Kabelbinders, was den Vorteil einer begrenzten Längenverstellbarkeit hat oder eines Klemm- oder Klammerverschlusses, ähnlich einer Metallklemme, in diesem Fall jedoch aus Kunststoff, oder einem einer sehr stramm schließenden Wäscheklammer ähnlichem Verschluss.

Eine weitere bevorzugte Weiterbildung des Penisextensionssystems sieht vor, den Eichelschlitten an seiner Unterseite direkt mit einem Klett- oder einem anderen Verschluss zu versehen, so dass sich der Eichelschlitten mit der gewünschten Zugkraft direkt an einer entsprechenden Stelle einer in diesem Fall mit dem Verschlussgegenstück, sei es das Flauschband eines Klettverschlusses oder das Gegenstück eines Druckknopfes, ausgestatteten Unterhose, befestigen lässt.

Eine weitere bevorzugte Weiterbildung der Vorrichtung zeichnet sich dadurch aus, dass das Befestigungselement zur Befestigung der Penisspitze dergestalt halbzylindermantetförmig ausgebildet ist, dass es an seiner bei Gebrauch dem Benutzer zugewandten Stirnseite offen ist, so dass ein Benutzer seinen Penis entlang der Längsachse in der Innenfläche des Befestigungselements platzieren kann, um ihn hinter der Eichel mit dem quer zur Längsachse verlaufenden, das Befestigungselement und den in ihr gebetteten Penis umschließenden, gepolstertem Band- oder Riemenverschluss zu fixieren. Während der Benutzung ist die geschlossene Außenfläche des Halbzylinders einem Benutzer zugewandt und befindet sich horizontal etwa in Höhe der Leistenbeuge. Die einem Benutzer zugewandte, halbkreisähnlich gewölbte Außenseite des Zylinders ist glatt und gleitbar ausgebildet und ermöglicht einem Benutzer das Hinsetzen ohne das er befürchten muss, durch scharfe Kanten oder Ecken hierbei behindert, oder in der Leistenbeuge verletzt zu werden.

Eine weitere bevorzugte Weiterbildung der Vorrichtung zeichnet sich dadurch aus, dass die nicht mit der Zugvorrichtung verbundene, gepolsterte Schlaufe welche als Widerlager gegen die Zugkraft an der Penisspitze wirkt, dehnbar ist. Die Auswahl an Befestigungsvorrichtungen ist wie bei der Befestigungsvorrichtung für das Zugmedium vielfältig und orientiert sich in erster Linie an den Kundenwünschen. Hervorzuheben ist jedoch auch hier der gewebeschonende und an seiner einem Benutzer zugewandten Seite, gepolsterte Hosenträgerclip oder Klemmverschluss, der den Vorteil bietet, unabhängig und ohne anzubringendes Gegenstück, an einer beliebigen Stelle der Bekleidung befestigt werden zu können.

Eine weitere bevorzugte Weiterbildung des erfindungsgemäßen Penisextensionssystems besteht darin; dass die als Befestigungsbasis für das Zugelement und das Widerlager dienende Bekleidung als Unterhose dergestalt ausgebildet ist, das sie um die vordere Mitte, auch als Schlitz oder Eingriff bekannt, wo sich während des Tragens der Penis und der Hodensack befinden, Befestigungsmöglichkeiten für das Zugelement und das Widerlagerelement aufweist, die in einem unterschiedlichen Abstand angeordnet und befestigt oder befestigbar sind, um dem Benutzer die Streckung des Penis in verschiedene Richtungen mit unterschiedlicher Zugstärke und unterschiedlichen Befestigungsvorrichtungen zu ermöglichen.

Eine weitere beispielhafte Ausführungsvariante des Penisextensionssystems besteht darin, dass die an der als Basiselement dienenden Hose angebrachten Befestigungspunkte für das Zugelement oder für das Widerlager auch für andere formgebende Anwendungen der Geschlechtsorgane genutzt werden. Denkbar ist eine PushUp-Funktion wie bei einem BH, indem ein Kissen unter dem Penis und dem Hodensack mittels der zu beiden Seiten befindlichen Befestigungspunkte gespannt wird, wodurch Penis und Hodensack unter der Oberbekleidungshose angehoben werden und für einen unbefangenen Betrachter der Eindruck eines größeren Volumens der Geschlechtsteile entsteht.

Eine weitere beispielhafte Variante des Penisextensionssystems besteht darin, dass die an der als Basiselement dienenden Hose angebrachten Befestigungspunkte für das Zugelement oder für das Widerlager auch für den Beischlaf vorbereitende Anwendungen genutzt werden können, indem der Penis, oder der Penis und der Hodensack an ihrer Basis zwischen zwei in die entgegengesetzte Richtung ziehende Widerlagerschlaufen oder Halteriemen platziert werden. Durch diese Maßnahme wird der Rückfluß des venösen Blutes aus den Schwellkörpern behindert und es entsteht eine länger andauerndere und kräftigere Erektion.

Eine weitere beispielhafte Weiterbildung des erfindungsgemäßen Penisexdensionssystems besteht darin, dass ein Benutzer die Möglichkeit hat, statt der Unterhose als Basis- oder Befestigungselement, auch ein beide Beine einzeln mit Bändern umschließendes, untereinander jedoch durch einen Steg verbundenes, bildlich formuliert, ein eine 8 bildendes Gurtsystem wählen kann. Die Gurte sind durch Längenverstellbarkeit dem Oberschenkelumfang eines Benutzers anpassbar und/oder aus einem dehnbaren Material gefertigt. Ein Benutzer steigt in diese beiden kreisförmigen Gurte wie in eine Hose und zieht sie über die Beine hoch nach oben bis in den Schritt. Er hat nun die Möglichkeit, seinen Penis in Richtung linkes oder rechtes Hüftgelenk zu strecken, indem er zuerst das Widerlager des, der gewünschten Streckriehtung abgewandten Beingurtes über den Penis oder über den Penis und den Hodensack streift. Anschließend befestigt er die Eichel in dem Eichelschlitten, der nicht mit dem Basiselement sondern mit dem Zugelement verbunden ist. Dann nimmt er das freie Ende des Zugelements und befestigt dieses mit einer ihm angenehmen Zugstärke mit der Befestigungsvorrichtung an der entsprechenden Stelle des anderen Beingurtes. Da beide, die Beine umschließenden Gurte, durch einen Steg verbunden sind, ist ein Verrutschen durch den entstehenden Zug ausgeschlossen.

Eine weitere beispielhafte Weiterbildung des erfindungsgemäßen Penisextensionssystems zeichnet sich dadurch aus, dass das Befestigungselement für den Penis aus mehreren Medien besteht. Ein mit seiner Unterseite mit Verlauf des Innenradius des Eichelschlitten verbundene Flachschlauch dient zur Aufnahme des den ursächlichen Bindedruck ausübenden wahlweisen Mediums. Dies kann ein dehnbarer Kabelbinder, ein schmales Leder- oder Kunststoffarmband, ein Klettband oder ein beliebiger Schnellverschluss sein, da die Haut mit diesem Element nicht in Berührung kommt. An seiner Innenseite und seitlich ist der Flachschlauch mit einem weichen Polsterprofil beschichtet. Der Erfinder hat entdeckt, dass diese Art der erfindungsgemäßen "nicht unmittelbaren Befestigung" der Eichel deren gute Durchblutung sicherstellt und ein "kalt- oder blauwerden" im Vergleich zum gattungsgemäßen Stand der Technik auch nach längerem Tragen ausbleibt. Warum dies so ist, vermag er nicht zu sagen, denn er ist kein Physiker oder Mediziner. Wahrscheinlich liegt es daran, dass das ursächlich schnürende Medium unabhängig in dem Flachschlauch gleiten kann, der völlig andere Härte- und Reibungseigenschaften als das weiche Beschichtungsmaterial und das innen laufende Befestigungsmedium hat.

Eine weitere beispielhafte Weiterbildung der Erfindung zeichnet sich dadurch aus, dass das Befestigungselement flexibel auf den individuellen Umfang des zu befestigenden Penis eingestellt werden kann. Dies wird dadurch erreicht, dass der im inneren Radius des Eichelschlittens befestigte Flachschlauch an seiner dem Eichelschlitten abgewandten Außenseite für den Verschluss des Bindemediums offen ist. Die innenliegende, weich beschichtete Seite des Schlauches schiebt sich, als sich verjüngende Schutzzunge, in den sich nun bildenden Ringverschluss und gewährleistet so einen flexiblen Innenradius.

Eine weitere beispielhafte Weiterbildung des Systems zeichnet sich dadurch aus, dass der Benutzer des Penisextensionssystems statt des Eichelschlittens als Befestigungsmedium in Verbindung mit dem Zugelement, auch einen Unterdruckbehälter als Befestigungsmedium in Verbindung mit dem Zugelement verwenden kann, um erfindungsgemäß gleichzeitig zur Penisstreckung eine starke Eicheldehnung durch Unterdruck vorzunehmen. Diese sollte jedoch auf 30 Minuten pro Anwendung begrenzt sein, um dann wieder eine normale Durchblutung der Eichel zu gewährleisten und das Risiko von sonst möglichen Gewebeschäden auszuschließen. Ein Benutzer pumpt mit beiliegender Ballpumpe durch das seitlich am Unterdruckzylinder befindliche Ventil die luft aus dem Zylinder. Durch den künstlich erzeugten Unterdruck strömt vermehrt Blut in die Eichel, welche sich dadurch enorm vergrößert und ausdehnt. Diese zeitweilig vermehrte Durchblutung und Dehnung soll das Missverhältnis zwischen Peniskörper und Eichel diesbezüglich ausgleichen und das Wachstum, insbesondere der Eichel begünstigen. Die vorliegende Erfindung ist nach vorliegendem Wissensstand die einzige Vorrichtung, die gleichzeitig das mechanische Langziehen des Penis und die durch Unterdruck erzeugte große Ausdehnung und Durchblutung der Eichel ermöglicht. Der Unterdruckzylinder weist hinter der Eichel, dem Körper eines Benutzers zugewandt, eine den anatomischen Voraussetzungen eines Nutzers vom Innenradius angepasste Verengung auf, durch die ein Benutzer seinen Penis einführt, und die seinem Penis zuverlässigen Halt gegen die Zugkräfte des an der Spitze befestigten Zugelements bietet. Um den Unterdruckzylinder zur Penisstreckung mit dem Zugelement verbinden zu können, weist dieser an seiner Spitze an der Außenseite, eine Befestigungsvorrichtung für das Zugelement auf.

Eine weitere beispielhafte Weiterbildung der Erfindung ist dadurch gekennzeichnet, dass der Unterdruckzylinder mittels arretierbarem Verlängerungsstück, welches teleskopähnlich umfänglich über den, bei Anwendung dem Körper eines Benutzers zugewandten Teil des Unterdruckzylinders passt, an seinem nicht über den Unterdruckzylinder geschobenen, bei Benutzung am Körper eines Benutzers anliegenden Ende, eine weiche in das Verlängerungsstück passende Rohrmuffe zur Abstützung am Körper eines Benutzers aufweist, durch die dieser seinen Penis einführt, verlängerbar ist. Ein Benutzer kann die übereinander geschobenen Zylinder in der gewünschten Gesamtlänge mit dem Arretierungsklemmbügel in ihrer Stellung festsetzen und hat so die Möglichkeit, seinen Penis einer kurzfristigen sehr intensiven Streckung durch Unterdruck auszusetzen oder durch ein genaues Messen der Penislänge unter Unterdruck, den Erfolg der bisherigen Streckung zu überprüfen.

Eine weitere beispielhafte Weiterbildung des Penisextensionssystems zeichnet sich dadurch aus, dass ein gegen Berührungen im Bereich der Leistenbeuge empfindlicher Anwender als Basis- oder Befestigungselement für das Zugelement für den Penis und für das Widerlagerelement für die Peniswurzel, statt der Bekleidung oder Unterhose, einen halbzylindermantelförmig gebogenen Korpus verwenden kann, der von seiner Länge den anatomischen Gegebenheiten eines Benutzers angepasst, den Bereich der Leistenbeuge mit den dort verlaufenden Blutgefäßen wie Bein-/Beckenarterie und des Venensterns zwischen Peniswurzel und Hüftknochen bzw, Oberschenkel zuverlässig überbrückt und somit jegliche Gefährdung dieser Blutgefäße ausschließt. An einer, einem Benutzer zugewandten Stirnfläche ist ein Basisring befestigt, durch den der Penis eines Benutzers einführbar ist, wobei in der Innenfläche des Korpus entlang dessen Längsachse der Eichelschlitten gleitbar angeordnet ist, auf dem die Eichel des Penis eines Benutzers entlang dessen Längsachse befestigbar ist und wobei der Eichelschlitten über mindestens ein Zugelement beaufschlagbar ist, das vom Eichelschlitten ausgeht und über die bei Gebrauch vom Benutzer abgewandte stirnfläche des Korpus auf die Außenfläche des Korpus umgelenkbar und in Abhängigkeit von der einzustellenden Zugkraft an einer Stelle auf der Außenfläche des Korpus befestigbar ist.

### Beschreibung der Figuren

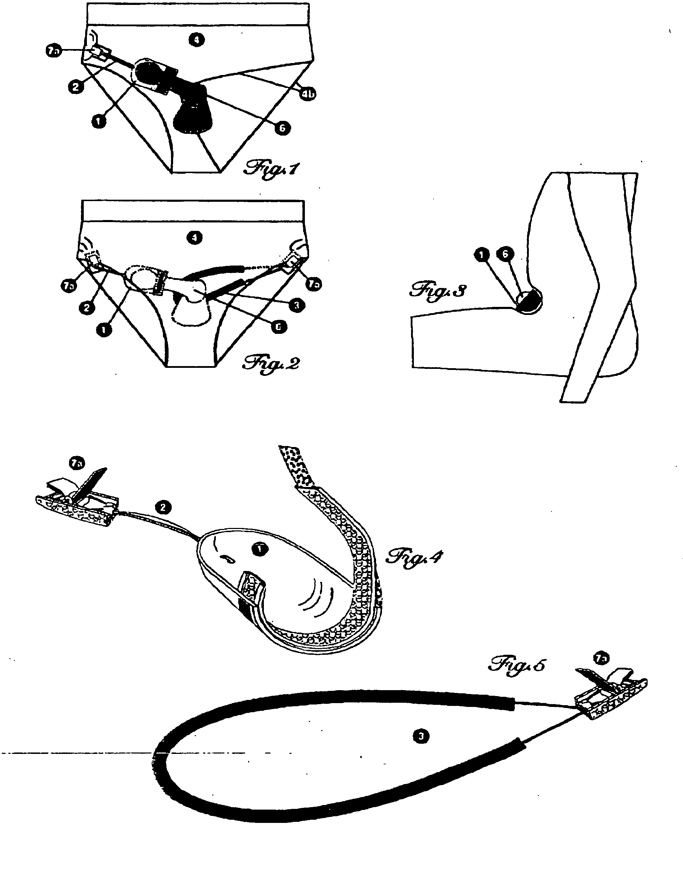

Die vorliegende Erfindung wird im Folgenden umfassend unter Bezugnahme auf die Figuren 1-25, die bevorzugte Ausführungsformen und unterschiedliche beispielhafte Anwendungsmöglichkeiten der Erfindung zeigen, näher erläutert. Die Darstellungen sind nicht maßstabgetreu.

Fig. 1 ist eine Ansicht der einfachsten Anwendung der Vorrichtung im gespannten Zustand, bei der auf die Detaildarstellung des Befestigungselements (1) Eichelschlitten, zu Gunsten der Übersichtlichkeit verzichtet wurde.

Ein Benutzer zieht eine Unterhose, die das Basiselement darstellt, an und streift den der gewünschten Zugrichtung des Penis gegenüberliegenden Beinbund (4b) über Penis und Hodensack, dass diese sich außerhalb der Unterhose befinden. Anschließend befestigt er den Penis (6) an dem Eichelschlitten (1) entlang dessen Längsachse, der noch nicht mit dem Basiselement (4), in diesem Fall einer Unterhose, sondern mit dem dehnbaren Zugelement (2) verbunden ist. Dann nimmt ziehend das freie Ende des dehnbaren Zugelements (2), das in diesem Fall als Befestigungsvorrichtung einen Hosenträgerclip (7a) aufweist, und verspürt die jetzt entstehenden Zugkräfte auf den Peniskörper. Er befestigt den mit dem dehnbaren Zugelement (2) verbundenen Hosenträgerclip (7a) nun mit einer ihm angenehmen Zugstärke, an einer entsprechenden Stelle der Unterhose (4). Die Zugvorrichtung übt nun einen permanenten, jedoch sich durch die hohe Flexibilität, den Bewegungen des Anwenders anpassenden Zug auf den Peniskörper (6) aus.

Fig. 2 zeigt die Anwendung der Vorrichtung, getragen unter einer herkömmlichen Unterhose, im gespannten Zustand unter Verwendung des Widerlagerelements (3). Ein Benutzer streift zuerst die Widerlagerschlaufe (3) über den Penis (6) oder über den Penis (6) und Hodensack zusammen und befestigt den hier beispielhaft als Befestigungsvorrichtung verwendeten an seiner Unterseite gepolsterten Hosenträgerclip (7a) von innen am Beinbund (4b) der Unterhose. Anschließend befestigt er den Penis (6) mit dem Riemen (1c) am Eichelschlitten (1a), entlang dessen Längsachse, der mit dem Zugelement (2) verbunden ist und zieht an dessen anderer Seite wo sich als Befestigungsvorrichtung (7) ein Hosenträgerclip (7a) befindet und befestigt diesen mit der von ihm gewünschten Zugstärke auf den Peniskörper von innen am anderen Beinbund (4b), der der Zug-richtung des Widerlagerelements (3) gegenüberliegt. Die Vorrichtung ist von einem Dritten, auch wenn ein Benutzer nur eine Unterhose oder Sporthose trägt, nun kaum wahrzunehmen.

Fig. 3 stellt die Lage des Eichelschlittens (1) während des Sitzens dar. Durch die glatte und runde Form des Eichelschlittens wird ein punktueller Druck, im Vergleich zum gattungsgemäßen Stand der Technik. vermieden. Ein Benutzer kann sich ungehindert und gesundheitlich ungefährdet setzen und bücken.

Fig. 4 und Fig. 5 stellen die Grundausstattung der Vorrichtung, zur Verwendung mit handelsüblicher Bekleidung oder Unterhose, bestehend aus Befestigungselement (1) für den Penis mit Zugelement (2) und Befestigungsvorrichtung (7a), sowie Widerlagerelement (3) mit Befestigungsvorrichtung (7a), dar.

Fig. 6 zeigt die Vorrichtung im gespannten Zustand unter Verwendung einer für das Penisextensionssystem konstruierten Funktionsunterhose (4). Der als Eingriff bekannte Stoffteil in der vorderen Mitte der Hose, wo sich der Penis (6) und der Hodensack eines Benutzers befinden, ist ausgespart, sodass die Hose in diesem Bereich nach außen offen zugänglich ist, jedoch mittels Druckknöpfen (4c und d) mit dem Eingriffteil abgedeckt werden kann. Zu beiden Seiten, sowie oberhalb und unterhalb dieses Bereiches, weist die Unterhose Befestigungsvorrichtungen (4c und d), hier in Form von Gegenstücken zu Druckknöpfen sowie Klettband (7i) als Gegenstück zu Flauschband (7i) auf. Ein Benutzer befestigt die Befestigungsvorrichtung (7i) des dehnbaren Zugelements (2), das in diesem Fall mit der Klettseite eines Klettverschlusses (7i) bestückt ist, an der dem gewünschten Zug entsprechenden Stelle des an der Hose angebrachten Flauschbandes. Ebenso verfährt er mit dem Widerlagerelement (3). Um den festen Halt des Hodensacks zu gewähren, ist diese Hose zur Aufnahme desselben mit einem Suspensorium ausgestattet.

Fig. 7 zeigt in Abb.1 bis Abb.3 das Kräfteschema der gegenübergestellten Vorrichtungen und in Abb.4 und Abb.5 das sich dazu unterscheidende Kräfteschema der vorliegenden Erfindung sowie in Abb. 6 und 7 beispielhaft Stehleiter und Anstellleiter.

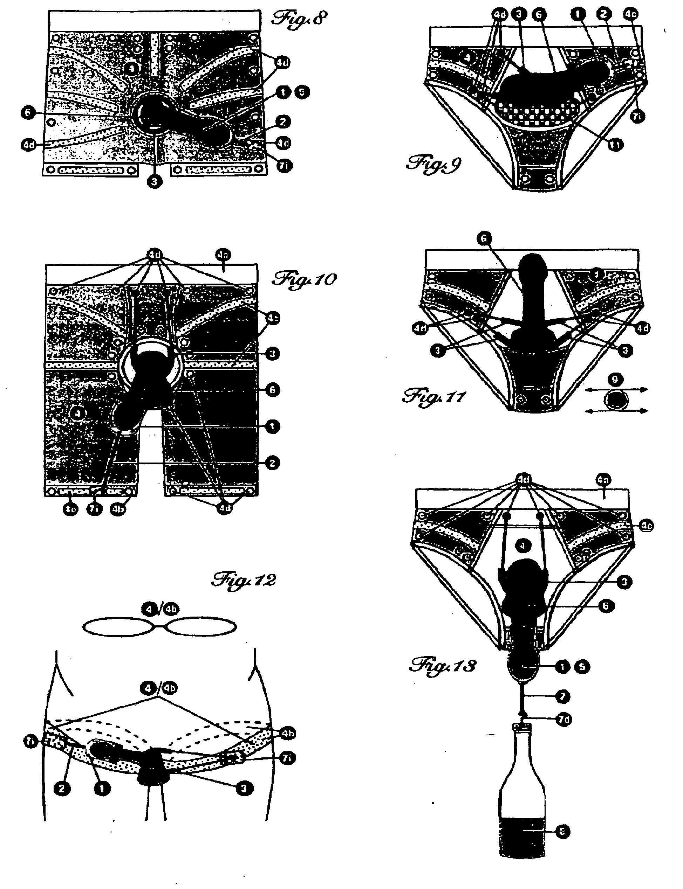

Fig. 8 ist eine Hose in Short-Form, bei der das zu allen Seiten wirksame Widerlager (3) für den Penis (6) durch einen eingefassten Ring gebildet wird. Die sternförmig aus Richtung der Peniswurzel verlaufenden beispielhaft verwendeten Klettbänder (7i) erlauben die Befestigung des Zugelements (2) in verschiedene Richtungen. Als mit dem dehnbaren Zugelement (2) verbundenes Befestigungselement für den Penis wird hier statt des Eichelschlittens, beispielhaft der Unterdruckbehälter (5) verwendet.

Fig. 9 stellt die Verwendung eines PushUp-Kissens (11) dar. Der Benutzer befestigt ein Kissen unterhalb von Penis und Hodensack mittels der zu beiden Seiten des Penis und Hodensack am Basiselement (4) befindlichen Befestigungspunkte (4 c und d). Er kann nun überdies noch eine Streckung vornehmen, ist aber durch das nach oben drückende Kissen auf die Richtungen rechte oder linke Hüfte eingeschränkt. In dieser Ansicht nimmt er eine Streckung in Richtung zur linken Hüfte, von ihm aus gesehen, vor.

Fig. 10 stellt die Streckung des Penis (6) mit einer knielangen Hose als Basiselement (4) dar. Das Widerlager (3) wird von zwei Haltepunkten (4d) mittig unterhalb des Hosenbundes (4a) gehalten und übt so den Gegendruck zu dem steil nach schräg unten Richtung Knie, zum rechten Beinbund (4b), vom Benutzer aus gesehen, befestigten dehnbaren Zugelement (2) aus.

Fig. 11 zeigt die Verwendung des Basiselements (4) mit zwei Widerlagern (3) als Hilfsmittel gegen die sogenannte erektile Dysfunktion, indem der Penis (6), oder der Penis und der Hodensack an ihrer Basis zwischen zwei in die entgegengesetzte Richtung ziehende Widerlager (3), die an jeweils zwei Haltepunkten (4d) des Basiselements (4) befestigt sind, platziert werden. Durch diese Maßnahme wird der Rückfluss des venösen Blutes aus den Schwellkörpern des Penis (6) behindert und es entsteht eine länger andauernde und kräftigere Erektion.

Fig. 12 zeigt die Verwendung des alternativen, nur die Beine in Form einer 8 umschließenden Basisteils (4/4b) unter Verwendung des Eichelschlittens als Befestigungselement (1) für den Penis. Beispielhaft wurde zur stufenlosen Befestigung des Zug- (2) und des Widerlagerelementes (3) Klettband verwendet. Da beide Beingurte durch einen Steg im Schritt miteinander verbunden sind, ist ein Verrutschen gegeneinander, ausgeschlossen.

Fig. 13 geht auf den Ursprung der Penisstreckung durch die Naturvölker zurück. Diese verwendeten bei Ihrer Streckung jedoch kein Widerlager (3). Das Widerlager (3) wird von zwei Haltepunkten (4d) am Basiselement (4), mittig unterhalb des Hosenbundes (4a) gehalten und übt so den Gegendruck zu dem steil nach unten, vom Benutzer aus gesehen, an einem Gewicht (8) zwischen seinen Beinen befestigten dehnbaren Zugelement (2), aus. Das Befestigungselement für den Penis ist in diesem Fall der Unterdruckzylinder (5). Das Gewicht (8) ist beispielhaft eine mit Sand gefüllte Flasche, an deren Schraubverschluss das Zugelement (2) mit einem Haken (7d) als Befestigungsvorrichtung (7) befestigt ist.

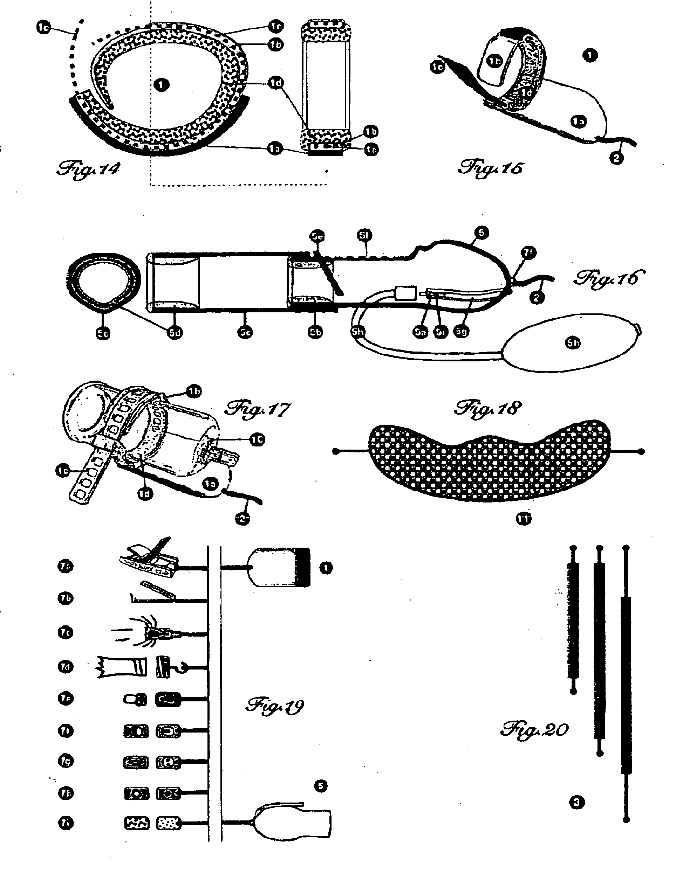

Figur 14 ist eine Darstellung des Befestigungselements (1) im Schnitt und von der Seite, das kreisförmig im inneren Radius des Eichelschlitten (1a) mit dem Flachschlauch (1b) der zur Aufnahme des Bindemediums (1c) dient, befestigt ist. An seiner Innenseite ist der Flachschlauch mit einem Polsterprofil (1d) beschichtet. Im, dem inneren Radius des Eichelschlitten gegenüberliegenden Bereich der Kreisform öffnet sich der Flachschlauch nach außen, um den Austritt des Bindemediums (1c) zu gestatten. Die mit dem Polsterprofil (1d) beschichtete Innenseite des Flachschlauchs (16) schiebt sich über das andere Ende des Flachschlauchs (1b) und schließt so das kreisförmige Befestigungselement nach innen. Das außen aus dem, kreisförmig an dem inneren Radius des Eichelschlitten angebrachten, Flachschlauch (1b) tretende Ende des Bindemediums (1c) wird nun ebenfalls mit seinem anderen Ende verbunden und mit einem von einem Benutzer als angenehm empfundenen Bindedruck verschlossen. Als Bindemedium kommen die verschiedensten Verschlussmöglichkeiten in Betracht, da sie mit der Haut nicht in Berührung kommen.

Figur 15 zeigt das Befestigungselement (1) bestehend aus Eichelschlitten (1a) mit Flachschlauch (1b) und darin verlaufendem beispielhaft verwendeten Klettverschluss als Bindemedium (1c).

Figur 16 zeigt einen Schnitt des beispielhaften zylindrisch geformten Unterdruckbehälters (5) zur Dehnung der Eichel bei gleichzeitiger Streckung des Penis durch die Verbindung mit dem Zugelement (2), welches mit dem Basiselement (4), oder mit dem Basiselement Korpus (14), oder mit einem Gewicht (8), oder mit dem Verlängerungsteil (5c) verbunden wird. Der vordere Teil zur Aufnahme der Eichel, ist entsprechend den anatomischen Gegebenheiten ausgeformt, während der hintere Teil, durch den ein Benutzer seinen Penis einführt, eine herausnehmbare Muffe (5b) aufweist, um verschiedene Penisgrößen aufzunehmen zu können. Der Anwender führt die Eichel vor die Manschette (5b) des Unterdruckbehälters (5). Nun pumpt er mit beiliegender Unterdruckpumpe die Luft über den parallel zur Eichelform verlaufenden zylinderförmigen, entlang seiner Längsachse zum Innenraum offenen Luftkanal, der an seinem bei Anwendung einem Benutzer zugewandten Ende einen Stutzen (5a) mit Ventil aufweist, aus dem Unterdruckbehälter (5). Dies hat den Vorteil, dass das punktuelle, verletzende Ansaugen einer Hautstelle ausgeschlossen werden kann. Durch den entstehenden Unterdruck wird die Eichel fest in den Unterdruckbehälter gezogen und dehnt sich durch das einströmende Blut enorm aus, bis sie den Behälter vollständig ausfüllt. Die herausnehmbare Verengung (5b) sorgt dafür, dass der Penis in dem Behälter fest fixiert ist und nicht zurückrutschen kann. Nun entfernt der Anwender die Unterdruckpumpe (5h) und befestigt den Unterdruckbehälter in der gleichen Weise wie den Eichelschlitten (1a) am Basiselement (4), um gleichzeitig zur Eichcldehnung eine Streckung des Peniskörpers zu erreichen. Das heißt, er zieht an dem, an der Spitze des Unterdruckbehälters (5) mit einer Ösmbekstigung (7f) angebrachten Zugelement (2) und befestigt dieses mit der Befestigungsvorrichtung Hosenträgerclip (7a), die am anderen Ende des Zugelements befestigt ist, an der dem gewünschten Zug entsprechenden Stelle des Basiselements (4) oder (14) oder an dem Gewicht (8). Zur Beendigung der Anwendung nach ca. 30 Minuten, betätigt er zum Auflösen des Vakuums den Knopf am Unterdruckventil (5i), um den Penis wieder aus dem Behälter zu ziehen. Zur Streckung ohne ein Basiselement oder zum Messen der Penislänge im erigierten Zustand, verlängert ein Benutzer den Unterdruckbehälter (5), indem er das ebenfalls zylindrische Verlängerungsteil (5c) über den im hinteren Bereich, bis zum Beginn der Eichelausbildung formgleichen, aber vom Durchmesser kleineren Unterdruckbehälter (5) schiebt und den Arretierungsklemmbügel (5e) bei der gewünschten Länge an der entsprechenden Arretierung (5f) am Unterdruckbehälter (5) einrastet.

Figur 17 zeigt eine perspektivische Ansicht eines mit dem Befestigungselement (1) mittels beispielhaft verwendeten dehnbarem Kabelbinder (1c) auf dem Eichelschlitten (1a) fixierten, beliebigen handelsüblichen Unterdruckbehälters (5). Dieser hat den Nachteil, dass der Lunstutzen im vorderen Teil, bei Erzeugung des Unterdrucks zu Druckstellen und Verletzungen an der Eichel des Anwenders führen kann.

Figur 18 stellt das beidseitig mit den Befestigungspunkten (4d) mit dem Basiselement (4) zu verbindende PushUp-Kissen (11) zum Anheben von Penis und Hodensack dar.

Figur 19 zeigt eine Auswahl bevorzugter Verbindungsmöglichkeiten (7a - 7i) zwischen dem Basiselement (4) und dem Zugelement (2), das auf seiner anderen Seite wahlweise mit dem Eichelschlitten (1a) oder mit dem Unterdruckbehälter (5) zur Aufnahme der Penisspitze verbunden ist.

Figur 20 zeigt am Basiselement (4) zu befestigende gepolsterte, als Widerlager (3) an der Peniswurzel zu verwendende, unterschiedlich lange Widerlagerelemente

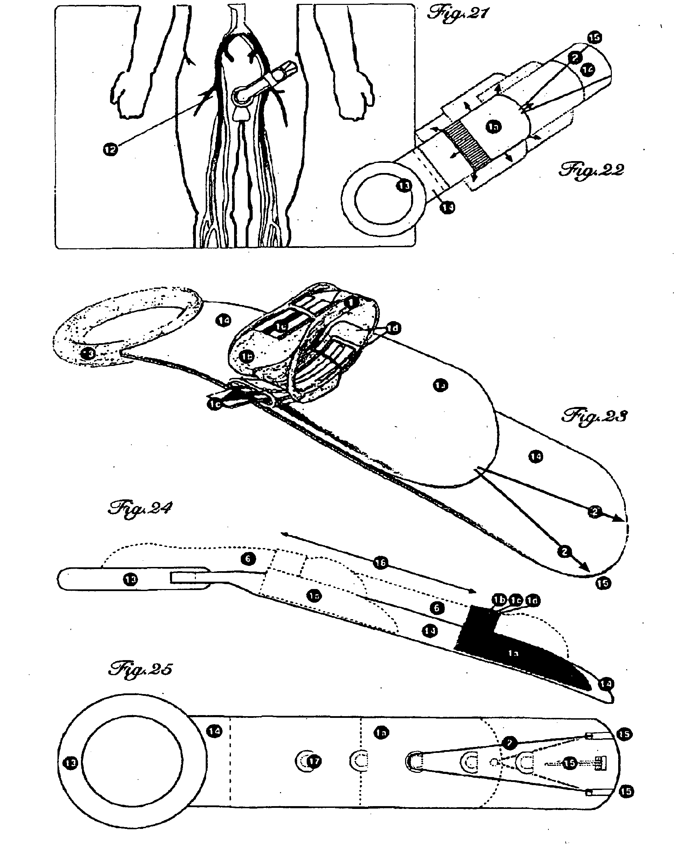

Figur 21 zeigt die Lage der in der Leistenbeuge verlaufenden Blutgefäße (12) auf. Die erfindungsgemäße Gestaltung des Basiselements Korpus (14) überbrückt diesen Bereich durch seine den anatomischen Gegebenheiten eines Nutzers angepasste Länge und ist bei Benutzung mit seiner geschlossenen, zylindermantelförmigen Längsseite dem Körper eines Benutzers zugewandt, so dass dieser vor scharfen Ecken und Kanten oder starren Teilen geschützt ist. Im Vergleich weisen alle bekannten gattungsmäßigen Penisextensionsgeräte in diesem Bereich gefährdende Teile auf.

Figur 22 zeigt den in dem halbzylindermantelförmigen Basiselement Korpus (14) gleitbar, entlang dessen Längsachse angeordneten, zu allen Seiten beweglichen Eichelschlitten (Ia), der von dem Zugelement (2), über die Führung der Korpusspitze (15) umgelenkbar, zur Befestigung auf dessen Außenseite gezogen wird.

Figur 23 ist eine perspektivische Gesamtansicht der Basiselements Korpus mit Eichelschlitten, bei der auf die Darstellung des Polsterprofils (1d, Fig. 14) des Befestigungselements (1) zu Gunsten der Übersichtlichkeit verzichtet wurde.

Der Anwender führt den Penis durch den Basisring (13), der fest mit dem Korpus (14) verbunden ist und hat jetzt beide Hände frei. In die eine Hand nimmt er nun den noch nicht mit dem Korpus verbundenen Eichelschlitten (1) und legt mit der anderen Hand seinen Penis entlang der Längsachse in den Eichelschlitten, dass die Eichel mit ihrer Spitze zur Befestigungsvorrichtung des an der abgerundeten Stirnseite des Eichelschlittens angebrachten Zugelements weist. Der kreisformige Riemenverschluss des Eichelschlittens (1b-1d) umschließt nun den Penisschaft hinter der Eichel und wird mit dem Bindeelement (1c) verschlossen. Mit einer Hand bewegt er nun den Basisring (13) zur Peniswurzel und hält ihn dort fest. Mit der anderen Hand nimmt er das Zugelement (2), an dem sich die, mittels Befestigungselement (1) auf dem Eichelschlitten fixierte Penisspitze befindet und zieht diese in der Führung des zylindemlantelförmigen Korpus (14) in Richtung Korpusspitze (15). Er verspürt die jetzt entstehenden Zugkräfte auf den Peniskörper. Er führt das Zugelement (2) über die Führung (15) an der Spitze des Korpus (14) und befestigt das Zugelement (2) mit einer ihm angenehmen Zugspannung an einem dieser Spannung entsprechenden, in der Korpusrückseite versenkten Befestigungspunkte (17). Das in sich geschlossene Kraftsystem übt nun einen permanenten, jedoch sich durch die hohe Flexibilität den Bewegungen eines Anwenders anpassenden Zug auf den Peniskörper aus.

Figur 24 ist eine Seitenansicht des Penisextensionsgerätes, bzw. eine Draufsicht des Anwenders von oben während des Benutzens, mit Lage des Penis (6) und der Eichel. In dieser Ansicht ist besonders gut die Funktion des Korpus (14) zur Überbrückung des Bereichs der hier verlaufenden großen Blutgefliße zu beurteilen.

Der beidseitige Pfeil (16) stellt den Zugbereich dar, dem der Peniskorpus ausgesetzt ist.

Figur 25 zeigt die geschlossene, während der Anwendung einem Benutzer zugewandte Außenseite des hibzylindermantelförmigen Basiselement Korpus (14), an dessen versenkten Befestigungspunkten (17), dass über die Zugelement-Führung (15) an der Spitze des Korpus, umgelenkbare Zugelement gespannt und befestigt wird.

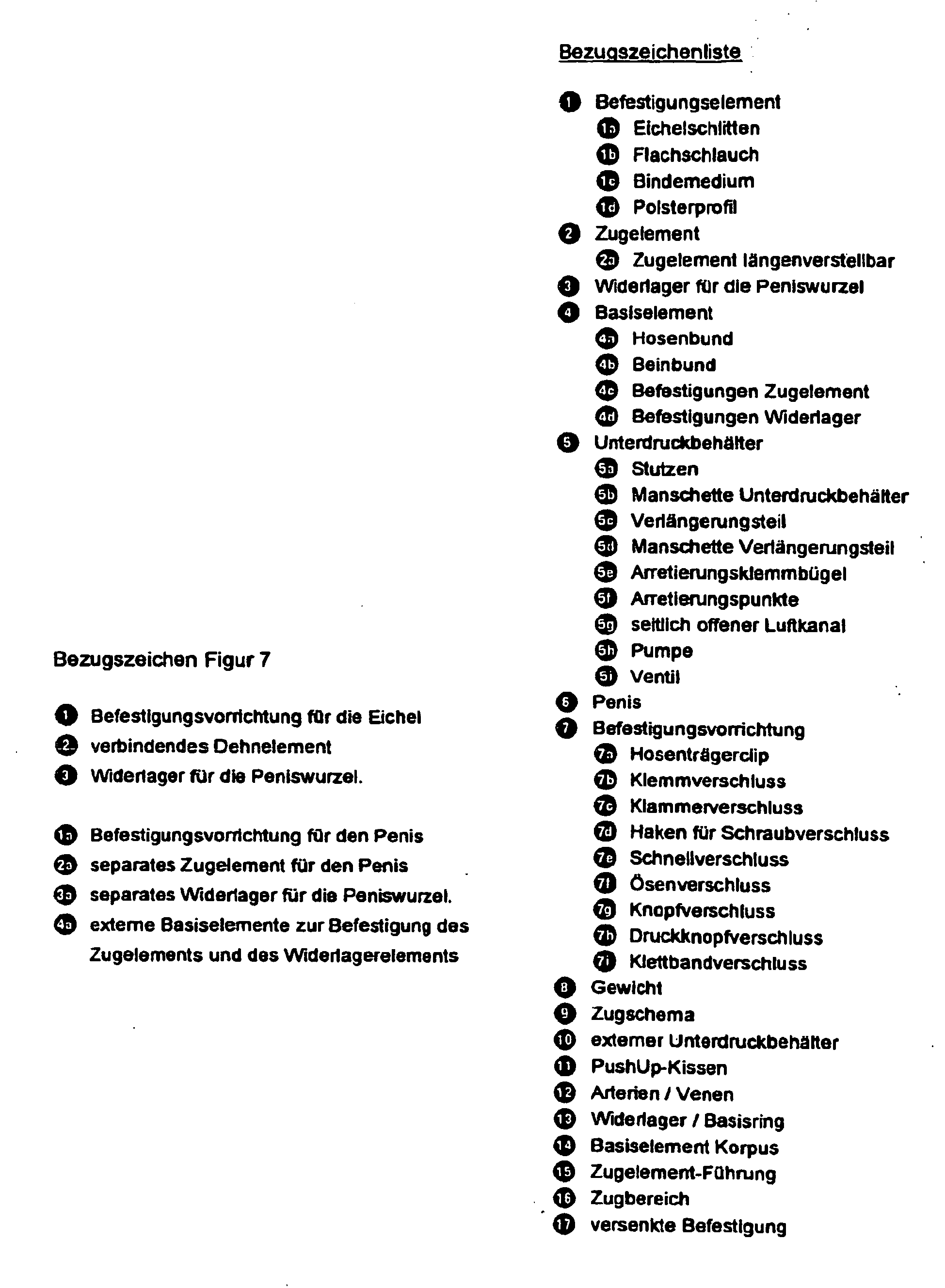

## Patentansprüche

1. In die Bekleidung eines Benutzers integrierbares, mobil zu tragendes Penisextensionssystem zur Vergrößerung des Penis und der Eichel mit einem, in Gebrauch die Eichel aufnehmenden, am Penisschaft befestigbaren Eichelschlitten (1), an dem ein Ende eines Zugelementes (2) befestigt ist und das andere Ende des Zugelementes mittels einer ersten Befestigungsvorrichtung (7) an einer ersten Stelle der Bekleidung (4) eines Benutzers lösbar befestigbar ist, wobei die erste Stelle der Bekleidung abhängt von der gewünschten Zugkrasft und Zugrichtung der über das Zugelement (2) auf den Penis zu übertragenden Zugkraft, **gekennzeichnet durch** ein Widerlager an der Peniswurzel eines Benutzers, das in Form einer um die Peniswurzel eines Benutzers verlaufenden Schlaufe (3), die an einer zweiten Stelle der Bekleidung (4) **durch** eine zweite Befestigungsvorrichtung (7) lösbar befestigbar ist, zur Erzeugung einer Zugkraft, die der Zugkraft am Penisschaft entgegen gerichtet ist.

2. Penisextensionssystem nach Anspruch 1,
**dadurch gekennzeichnet,**
**dass** das Zugelement (2) elastisch dehnbar ist.

3. Penisextensionssystem nach Anspruch 1,
**dadurch gekennzeichnet,**
**dass** das Zugelement (2) längenverstellbar ist.

4. Integriertes Penisextensionssystem nach einem oder mehreren der vorhergehenden Ansprüche, dadurh gekennzeichnet,
dass ein halbzylindermantelförmiger Eicheischlitten (1) für den Penis, diesen in seinem vorderen Bereich in seinem Umfang radial entlang der Längsachse teilweise umgibt und die Penisspitze quer zur längsachse des Penis, teilweise umgibt.

5. Integriertes Penisextensionssystem nach einem oder mehreren der vorhergehenden Ansprüche,
**dadurch gekennzeichnet,**
**dass** die durch Zugelement (2) und durch das Widerlager (3) am Penis eines Benutzers angreifenden Kräfte einander entgegen gerichtet sind.

6. Integriertes Penisextensionssystem nach einem oder mehreren der vorhergehenden Ansprüche,
**dadurch gekennzeichnet,**
**dass** die erste und/oder die zweite Befestigungsvorrichtung (7) als Stoffclip, bekannt als Hosentragercup (7i) ausgebildet ist.

7. Integriertes Penisextensionssystem nach einem oder mehreren der vorbergebenden Ansprüche,
**dadurch gekennzeichnet,**
**dass** der Penis eines Benutzers durch mindestens einen am Eicheischlitten (1a) befestigten, gunähnlichen Verschluss (1b-1d) befestigbar ist.

## Claims

1. Penis extension system which can be integrated in the clothes of a user and worn mobile, for enlargement of the penis and glans with a glans slide (1), which receives the glans and can be fixed on the penis shaft, on which one end of a pull element (2) is fixed. The other end of the pull element is fixed detachable by means of a first fixation device (7) on a first position of the clothes (4) of a user. This first position of the clothes depends on the required pulling force and pulling direction, which should be applied by the pull element (2) onto the penis. It is identified by a thrust bearing on the root of the penis of a user, which is opposed to the pulling force on the shaft (in form of a loop around the penis root of a user (3), which can be fixed detachable on a second position of the clothes (4) by a second fixation device (7) for production of a pulling force.

2. Penis extension system acc. to claim 1, **characterized by** the fact, that the pulling element (2) is elastically flexible.

3. Penis extension system acc. to claim 1, **characterized by** the fact, that the pulling element (2) is length adjustable.

4. Integrated penis extension system acc. to one or several of the above mentioned claims, **characterized by** the fact, that a glans slide in form of a profile cylinder (1) surrounds partially the penis in its front area along the longitudinal axis and partially the tip of the penis across the longitudinal axis.

5. Integrated penis extension system acc. to one or several of the above mentioned claims, **characterized by** the fact, that forces by the pulling element (2) and the thrust bearing (3) on the penis of a user are opposed to each other.

6. Integrated penis extension system acc. to one or several of the above mentioned claims, **characterized by** the fact, that die first and / or second fixation device (7) are formed as fabric clip, known as suspender clip (7i).

7. Integrated penis extension system acc. to one or several of the above mentioned claims, **characterized by** the fact, that the penis of a user can be fixed on at least one belt similar lock (1a) which is fixed on the glans slide.

## Revendications

1. Système d'extension du pénis, qui peut être intégré dans les vêtements de l'utilisateur et porté mobil pour grandissement du pénis et du gland par une glisse de gland (1), qui acceuillit le gland et peut être fixé à la tige du pénis. Un bout d'un élément de tirage (2) est fixé à la glisse de gland. L'autre bout de l'élément de tirage est fixé détachable à l'aide d'un premier dispositif de fixation (7) à une première position des vêtements (4) d'un utilisateur. La première position des vêtements dépend à la force et direction de traction demandée, qui est transférée au pénis par l'élément de tirage (2), **caractérisé par** une butée à la racine du pénis en forme d'une dragonne (3), qui est fixée détachable à une seconde position des vêtements (4) par un deuxième dispositif de fixation (7) pour la production d'une force de traction, qui est opposée à la force de traction à la tige du pénis.

2. Système d'extension du pénis selon revendication 1, **caractérisé par le fait, que** l'élément de traction (2) est élastiquement flexible.

3. Système d'extension du pénis selon revendication 1, **caractérisé par le fait, que** la longueur de l'élément de traction (2) est ajustable.

4. Système d'extension du pénis intégré selon un ou plusieurs des revendications susmentionnés, **caractérisé par le fait, qu'**un chariot de gland en forme de semi-cylindre (1) pour le pénis, entoure cela dans sa rogion avante radialement partiellement le long de l'axe longitudinal et le bout du pénis en travers de l'axe longitudinal du pénis.

5. Système d'extension du pénis intégré selon un ou plusieurs des revendications susmentionnés, **caractérisé par le fait, que** die les forces, qui ont effet au pénis par l'élément de traction (2) et par la butée (3) sont opposées.

6. Système d'extension du pénis intégré selon un ou plusieurs des revendications susmentionnés, **caractérisé par le fait, que** le premier et / ou le deuxième dispositif de fixation (7) est formé comme clip de bretelles (7i).

7. Système d'extension du pénis intégré selon un ou plusieurs des revendications susmentionnés, **caractérisé par le fait, que** le pénis d'un utilisateur peut être fixé par une fermeture pareille à une courroie(1b-1d), qui est fixé au chariot de gland (1a).
